# EUROPEAN PATENT APPLICATION

(11) **EP 1 629 845 A2**
(43) Date of publication of application: **01.03.2006**
(21) Application number: 05111297.7
(22) Date of filing: 30.10.2001
(51) Int. Cl.: A61K 31/724

(54) **Use of cortisol-sequestering agents for the treatment of hypercortisolaemia related disorders**

(30) Priority: 02.11.2000 EP 00309725
(62) Divisional of application: 01992568.4
(71) Applicant: Akzo Nobel N.V., 6824 BM Arnhem (NL)
(72) Inventor: Zhang, M., Lanarkshire Newhouse, ML1 5SH (GB); Hill, D., Lanarkshire Newhouse, ML1 5SH (GB); Rees, D., 41268 Gothenburg (SE)
(74) Representative: Kraak, Hajo

(57) **Abstract**

The invention relates to the use of sequestering agents for the preparation of a medicament for the treatment of hypercortisolaemia related disorders, especially for the treatment of major depression; and to pharmaceutical compositions comprising a cortisol-sequestering agent.

## Description

The invention relates to the use of sequestering agents for the preparation of a medicament for the treatment of hypercortisolaemia related disorders, especially for the treatment of major depression.

One of the most consistent findings in psychiatry is that patients with major depression present with hypercortisolaemia (Holsboer, F. et al., *Cortisol, 11-deoxycortisol and ACTH concentrations after dexamethasone in depressed patients and healthy volunteers.* Psychiatry Res. 11, 15-23, 1984)). This hypercortisolaemia can reach an order of magnitude and indicates a gross disturbance in the negative feedback mechanisms that normally strongly regulate the endocrine stress HPA (hypothalamo-pituitary-adrenal) axis. Although not all depressed patients have manifest hypercortisolaemia, patients who suffer the most severe types of monoamine-resistant depression are in majority those in whom high levels of the stress hormone cortisol are apparent. Further, in almost all cases they exhibit a clear inability to switch off endogenous cortisol release following exogenous challenge with the potent synthetic glucocorticoid dexamethasone (the so-called dexamethasone non-suppressors) (Gold P.W., et al., *Clinical and biochemical manifestations of depression: relation to neurobiology* of *stress.* New England J. Med. 319, 413-420, 1988). This 'sub-group' of severely compromised patients are most often the ones in whom depression becomes a life-threatening illness that warrants hospitalisation. A prompt treatment strategy like electroconvulsive therapy (ECT) may be necessary in such patients.

Another condition in which high cortisol levels are reported as a result of adrenal gland malfunction (due to a pituitary tumour or a secondary tumour, both producing the cortisol secretagogue ACTH) is the Cushing's syndrome. The depressive symptoms associated with Cushing's disappear relatively quickly once the underlying cause of the disease has been treated, with the return of cortisol levels to normal. Such treatment may involve removal of the offending tumour or treatment with cortisol synthesis inhibitors such as metyrapone, ketoconozole, or aminoglutethimide (Murpy, B.E.P., *Steroids and Depression.* J. Steroid Biochem & Mol. Biol. 38, 537-558, 1991).

Similarly, relatively recent clinical trials have demonstrated that cortisol synthesis inhibitors can be used to ameliorate depressive symptoms in severe, treatment-resistant non-Cushing depressives (Murphy, B.E.P., *Neuroendocrine responses to inhibitors of steroid synthesis in patients with major depression resistent to antidepressant therapy.* Can. J. Psych. 43, 279-286, 1998; see also US Patent 4,814,333 (Ravaris, C.L.): *Method for treatment of hypercortisolemic, depressed patients.).*

Drawbacks of the use of cortisol synthesis inhibitors to lower plasma cortisol levels are their high toxicity and their relatively low degree of selectivity for inhibition of cortisol synthesis versus synthesis of other endogenously manufactured steroids (such as mineralocorticoids and sex steroids) which can result in adrenal insufficiencies. A further serious disadvantage is that the onset of therapeutic effect of these cortisol synthesis inhibitors is as long as that observed with classical antidepressants (e.g., several weeks).

A need therefore exists for alternative means to treat patients suffering from a hypercortisolaemia related disorder, which means have a fast onset of the therapeutic effect and which means are devoid of excessive undesired side-effects.

The present invention accordingly provides for cortisol-sequestering agents to lower blood cortisol levels and to treat hypercortisolaemia related disorders. By parenteral administration of the cortisol-sequestering agent according to the invention a safe and acute way of lowering cortisol in a patient is provided.

One aspect of the invention relates to the use of a cortisol-sequestering agent for the manufacture of a medicament for the treatment of hypercortisolaemia related disorders. Preferred hypercortisolaemia related disorders are major depression, Cushing's syndrome, schizophrenia and severe anxiety disorders. More preferred is the use of a cortisol-sequestering agent according to the invention in the treatment of major depression, and especially so of major depression that does not respond adequately to the treatment with monoamine-based antidepressants, e.g., specific serotonin reuptake inhibitors (SSRIs).

Hypercortisolaemia is a relative term. Just as the normal cortisol plasma level in humans ranges from 140-552 nmoles/L (Greenspan, F.S. 1994 "Basic & Clinical Endoclinology; 4^{th} Edition, Norwalk, Appleton-Lange) plasma cortisol levels can vary widely from depressed patient to depressed patient, although they generally peak in excess of 800 nmoles/L, and they vary with time of day. Depressed patients, unlike Cushing's patients who exhibit constantly high levels of cortisol, maintain some degree of circadian fluctuation, albeit somewhat flattened. However, one cortisol level that is widely accepted as a diagnostic tool is the dexamethason suppression level. In this ubiquitously utilized 'test' for depression, 1 mg dexamethasone is given at bedtime and cortisol levels drawn at 4 p.m. the next day (normally the nadir of secretion). A dexamethason non-suppressor is classified as a patient that exhibit a 4 p.m. level of 136 nmoles/L or more. It is estimated therefore, that the therapeutic window in which a cortisol sequestering agent would be required to operate encompass plasma cortisol levels of 100-1000 nmoles/L.

The term sequestering agent (or sequestrant), as used in the present invention, means any organic compounds which can engage in host-guest complex formation with the steroid cortisol. The sequestrant acts as the host molecule, with cortisol being the guest molecule. The specific molecular complex, the host-guest complex, is defined as an organised chemical entity resulting from the association of two or more components held together by noncovalent intermolecular forces.

Sequestrants for cortisol may be host molecules selected from various classes of, mostly cyclic, organic compounds which are known for their ability to form complexes with various organic guest molecules, especially steroidal compounds, in aqueous solution, e.g. cyclic oligosaccharides, such as cyclodextrins, cyclophanes, cyclic peptides, calixarenes, crown ethers and aza crown ethers. Structures and chemistry of these compounds are well documented (*Comprehensive Supramolecular Chemistry,* Volumes 1-11, Atwood J.L., Davies J.E.D., MacNicol D.D., Vogtle F., eds; Elsevier Science Ltd., Oxford, UK, 1996).

Preferred sequestering agents for use with the present invention are cyclic oligosaccharides. Examples of cyclic oligosaccharides suitable for use with the invention are the cyclodextrins, a catagory of naturally occcurring cyclomaltooligosaccharides, the cyclomannins (5 or more α-D-mannopyranose units linked at the 1,4 positions by α linkages), the cyclogalactins (5 or more β-D-galactopyranose units linked at the 1,4 positions by β linkages), the cycloaltrins (5 or more α-D-altropyranose units linked at the 1,4 positions by α linkages), each of which are capable of forming guest-host complexes.

Particularly preferred are sequestering agents for cortisol selected within the class of cyclodextrins derivatives: 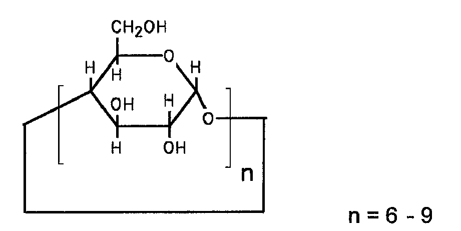
Cyclodextrins are cyclic molecules containing six or more α-D-glucopyranose units linked at the 1,4 positions by α linkages as in amylose. As a consequence of this cyclic arrangement, the cyclodextrins exist as conical shaped molecules with a lipophilic cavity which can attract guest molecules whilst the outside is more hydrophilic and water-soluble. Cyclodextrins composed of six, seven, eight and nine glucopyranose units are commonly known as α-, β-, γ- and δ-cyclodextrins respectively.

Both the native cyclodextrins (α, β, γ) which are prepared by enzymatic degradation of starch, and chemically modified cyclodextrins, have already been found, by virtue of their ability to form host-guest complexes, numerous applications especially in the pharmaceutical field (Stella and Rajewski: Pharmaceutical Research, 14, 556-567, 1997). The majority of these applications are in the pharmaceutical formulations of drugs (see for instance Uekama et al. "*Cyclodextrin Drug Carrier Systems*" Chemical Reviews 1998, 98, 2045-2076; Zhang, M. and Rees, D.C.: Exp. Opin. Ther. Patents, 9, 1697-1717, 1999).

Several known cyclodextrins (CD) form inclusion complexes with cortisol. Such an inclusion complex between CDs and cortisol, when formed in vivo, will reduce the availability of cortisol for its biological receptors and could therefore normalise HPA system. Furthermore, it is anticipated that CDs would increase the renal clearance of cortisol, resulting in an effective reduction of plasma levels of free cortisol.

Table 1 lists the association constants of selected CD-cortisol complexes for which 1:1 stoichiometry in water has been reported as well as for the complex with the novel cyclodextrin derivative per-6-deoxy-per-6-(2-carboxyethyl)thio-γ-cyclodextrin.

**Table 1: Association Constants of 1:1 Cyclodextrin:Cortisol Complexes**

| **Cyclodextrin** | **Association Constant (K**_{**a**}**, M**^{**-1**}**)** |
|---|---|
| α-CD | 57 |
| β-CD | 1720-3000 |
| γ-CD | 2240 |
| Hydroxypropyl-β-CD | 900-1000 |
| Per-2,6-methyl-β-CD | 5910 |
| Per-6-acetyl-β-CD | 3920 |
| Per-6-deoxy-per-6-(2-carboxyethyl)thio-γ-cyclodextrin | 40000* |

| | |
|---|---|
| *: measured by microcalorimetry | |

An important criterium for the selection of suitable sequestering agents for cortisol will be their selectivity of binding cortisol in comparison with other endogenous steroids, such as testosterone, estradiol, progesterone, cholesterol, etc. Although cholesterol occurs at relatively high plasma concentration, this steroid differ appreciably from cortisol in chemical structure. Cholesterol has a cis-configuration between the fused A-ring and B-ring and thus exists in a "bent" or "L-shape" conformation. In contrast, cortisol has a *trans*-configuration between the fused A-ring and B-ring and thus exists in a more "linear" conformation. Cortisol has also the characteristic of 3 hydroxyl functions in the molecule. It will be appreciated by the skilled person that such structural between the steroids form the basis on which selective sequestering agents can be designed and selected.

In order to form an inclusion complex with cortisol, the host molecule must have a size and shape which will allow it to at least partially encapsulate the steroid in its cavity. It is apparent from Table 1 that the cavity of α-CD is too small for sizeable encapsulation of cortisol. Therefore the binding affinity of α-CD is much weaker than that of the larger β- and γ-CDs. Since the depth of a CD cavity (~8 Å) is significantly shorter than that required to fully encapsulate all four rings of cortisol, extending the cavity depth, e.g., by per-substitution of primary and/or secondary hydroxyl groups, would be a method to increase affinity.

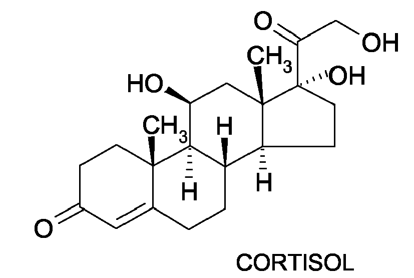

The interaction between a sequestering agent and cortisol can be analyzed by physical methods such as nuclear magnetic resonance spectroscopy (nmr) (Fielding, L.: Tetrahedron, 56 (34), 6151-6170, 2000) and microcalorimetry (Stoedeman M. and Wadsoe I.: Pure Appl. Chem., 67(7), 1059-68, 1995). These techniques also allow one to establish the selectivity of the binding to cortisol in comparison with the other endogenous steroidal compounds.

The sequestering agents for use in the invention are administered parenterally. The injection route can be intravenous, subcutaneous, intradermal, intramuscular, or intraarterial. The intravenous route is the preferred one. The exact dose to be used will neccessarily be dependent upon the needs of the individual subject to whom the medicament is being administered. Extracorporal application of the sequestering agent for cortisol, for instance by mixing of the sequestering agent with the blood during dialysis or during plasmapheresis, is also contemplated.

Mixed with pharmaceutically suitable auxiliaries and pharmaceutically suitable liquids, e.g. as described in the standard reference, Gennaro et al., Remington's Pharmaceutical Sciences, (18th ed., Mack Publishing Company, 1990, Part 8: Pharmaceutical Preparations and Their Manufacture; see especially Chapter 84 on "Parenteral preparations, pp. 1545-1569; and Chapter 85 on "Intravenous admixtures", pp. 1570-1580) the sequestering agents can be applied in the form of a solution, e.g. for use as an injection preparation.

Alternatively, the pharmaceutical composition may be presented in unit-dose or multidose containers, for example sealed vials and ampoules, and may be stored in a freeze dried (lyophilised) condition requiring only the addition of the sterile liquid carrier, for example, water prior to use.

The invention further includes a pharmaceutical formulation, as hereinbefore described, in combination with packaging material suitable for said composition, said packaging material including instructions for the use of the composition for the use as hereinbefore described.

The invention is further illustrated in the following examples.

### Example 1. 6-per-deoxy-6-per-(2,3-dihydroxypropylthio)-γ-cyclodextrin (Org 26276).

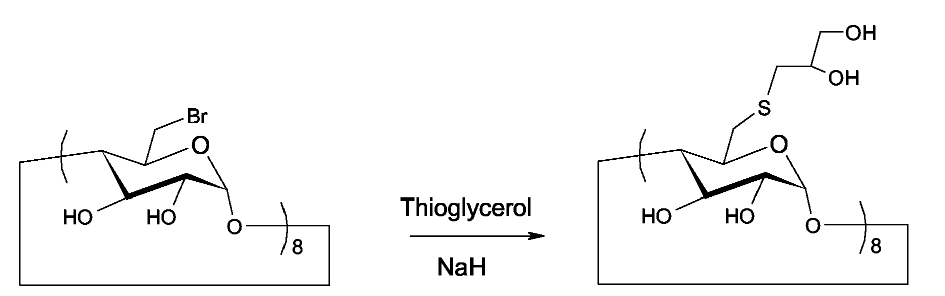

A 60% sodium hydride dispersion (450 mg) was washed oil free with heptane and then suspended in dry dimethylformamide (15 ml). A solution of thioglycerol (1.0 ml) in dimethylformamide (10 ml) was added slowly and the mixture stirred for 15 min. under nitrogen. *Per*-6-deoxy-*per*-6-bromo-γ-cyclodextrin (1.8 g: Gorin B.I., Riopelle R.J., Thatcher G.R.J. Tetrahedron Lett. 1996, 37, 4647-4650) was added and the mixture stirred at 90°C for 3h. Acetone was added to the cooled reaction mixture and the resulting solid filtered off, dissolved in water (60 ml) and dialysed for 6h to remove sodium salts. Concentration of the aqueous solution followed by precipitation of the cyclodextrin thioglycerol derivative by addition of acetone gave a buff coloured solid, 1.55 g (77% yield). Purity 99.6% by HPLC-ELSD).
¹H NMR (D₂O) : δ, 2.71 (8H, m, H-7a), 2.81 (8H, m, H-7b), 2.95 (8H, m, H-6a), 3.20 (8H, m, H-6b), 3.45-3.52 (16H, m, H-9a, H-4), 3.55-3.66 (16H, m, H-9b, H-2), 3.8-3.9 (16H, m, H-8, H-3), 3.95 (8H, m, H-5), 5.11 (8H, H-1).
¹³C NMR (D₂O): 34.1, 34.3 (C-6), 36.15, 36.2 (C-7), 64.80, 64.83 (C-9), 71.2, 71.3 (C-8), 71.8 (C-5), 72.2 (C-2), 73.0 (C-3), 83.6 (C-4), 101.7 (C-1).
MS (ESI), (M-H)- 2017 (C₇₂H₁₂₈O₄₈S₈).

### Example 2. Selection of a cortisol-sequestering agent.

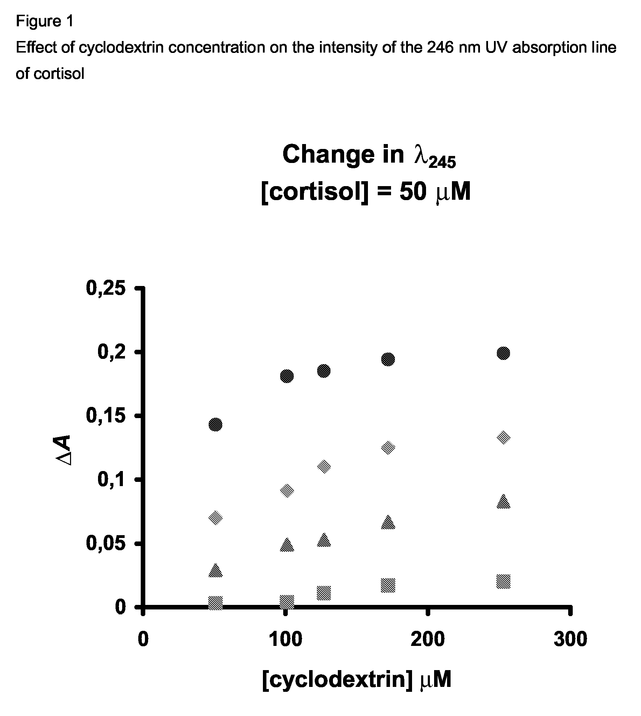

UV-visible spectroscopy was used to monitor the complex formation between cortisol and a library of cyclodextrin derivatives. The UV spectrum of cortisol contains an absorption band having a maximum at 246 nm. The intensity of the UV line at 246 nm decreases upon complexation of cortisol by addition of a cyclodextrin host molecule. The decrease in the absorbance of an aqueous solution of the guest molecule (cortisol) upon addition of a typical host molecule (like a cyclodextrin derivative) is taken as evidence that the guest molecule has moved from the polar aqueous environment to the usually less polar environment of the interior of the host molecule. The methodology is well established in the field of supramolecular chemistry (Tsukube, H. et al. In *Comprehensive Supramolecular Chemistry: Physical Methods in Supramolecular Chemistry.* Davies JED, Ripmeester JA, (Eds) Elsevier, Oxford, 1996: Vol 8, pp 425-483).
The association constants for complex formation are determined by performing a titration of cyclodextrin into cortisol and recording the UV spectrum as a function of the cyclodextrin/steroid ratio. The change in absorption of cortisol at 246 nm (Δ A) upon stepwise addition of cyclodextrin is recorded. The best fit line to the data give *K*ₐ for the cortisol-cyclodextrin complex (Liu Y, Li, B, You C-C, Wada, T, Inoue Y. J. Org. Chem., 2001, 66, 225-232). The UV methodology provides a fairly good estimate of the association constant when this constant is below 10⁵ M⁻¹. The results used for the determination of the association constant for the binding of cortisol to γ-cyclodextrin, β-cyclodextrin and to 2 γ-cyclodextrin derivatives are shown in Figure 1.
Description of the figure 1: Effect of cyclodextrin concentration on the intensity of the 246 nm UV absorption line of cortisol.
(■) γ-cyclodextrin, *K*ₐ = 2,300 M⁻¹;
(▲) β-cyclodextrin, *K*ₐ = 5,300 M⁻¹;
(◆) 6-*per*-deoxy-6-*per*-(2-carboxyethyl)thio-γ-cyclodextrin, *K*ₐ = 30,000 M⁻¹;
(●) 6-*per*-deoxy-6-*per*-(2-hydroxyethylthio)-γ-cyclodextrin, *K*ₐ ca. 10⁵ M⁻¹.

The data indicate that the native cyclodextrins have a relatively poor affinity to interact with cortisol. A number of cyclodextrin derivatives with marked enhanced affinity for cortisol were identified in the library having *K*ₐs greater than can be reliably measured by UV spectroscopy. This is because the chromophore in cortisol is relatively weak and instrument signal to noise issues prevented the preparation of sufficiently dilute solutions. The *K*ₐs for three of the cyclodextrin derivatives (see Table 2), i.e. 6-*per-*deoxy-6-*per*-(2,3-dihydroxypropylthio)-γ-cyclodextrin, 6-*per*-deoxy-6-*per*-(2-hydroxyethylthio)-γ-cyclodextrin and 6-*per*-deoxy-6-*per*-(3-carboxypropyl)thio-γ-cyclodextrin, are significantly greater than 10⁵ M⁻¹ and may even be ca. 10⁶. The high cortisol binding constant for these γ-cyclodextrin derivatives indicates these derivatives to be potentially very active cortisol sequestering agents.
An alternative, but more laborious, method for the determination of the cortisol association constant is by isothermal titration calorimetry (see Wiseman et al.
Analytical Chem. 1989, 179, 131-137), which can be used to determine the high Ka's more accurately.

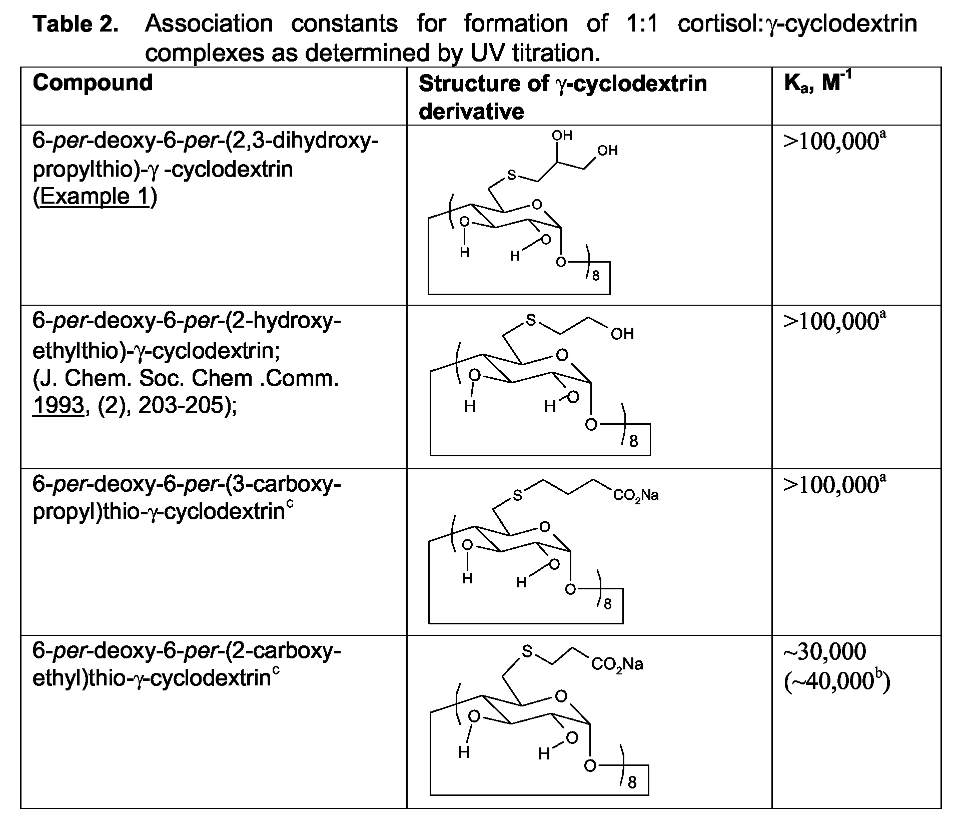

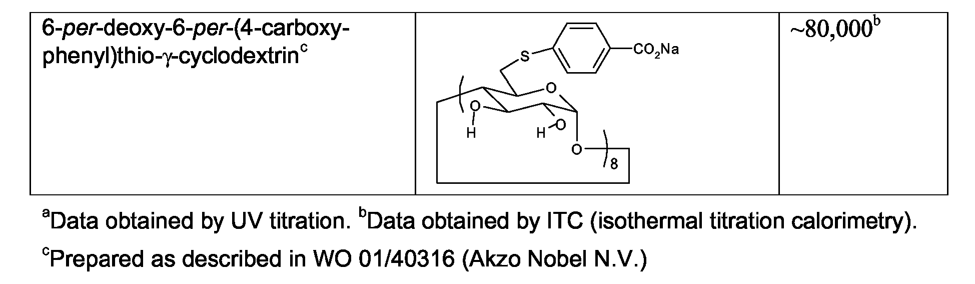

### Example 3.

### A: Urinary excretion of exogenous cortisol in the rat.

Rats do not have endogenous cortisol production. Two groups of four rats (Lister hooded rats, 300-320 g) were administered (intravenously) either 40µg/kg of cortisol alone (Group I) or cortisol followed after 15 minutes by 6-*per*-deoxy-6-*per*-(2,3-dihydroxypropylthio)-y -cyclodextrin (Org 26276) as a single intravenous dose of 20 mg/kg (Group II). Each rat was placed in a metabolic cage for the collection of urine. Urine samples were collected for the first 5 hours and thereafter for the remainder of 24 hours The collected urine samples were subjected to HPLC-MS (high pressure liquid chromatograpy on a Phenomenex Luna (50x2 mm; 5 µm) column coupled to a PE Sciex API 3000 Mass Spectrometer). The limit of quantitation for cortisol in urine was 2.5 ng/ml and 10 ng/ml in plasma.
The bulk of the urinary cortisol excretion occurred in the first 5 hours and there was no meaningful excretion of cortisol in the 5-24 hour period. The mean cortisol level in the urine collected in the first 5 hours from the Group I rats was 10.5±7.8 ng/ml, whilst the mean cortisol level in the urine of the Group II rats proved to be 111±78 ng/ml. These results demonstrate that Org 26276 enhanced the excretion of cortisol following intravenous bolus administration approximately 10-fold compared to the control group that received cortisol but not the cortisol-sequestering agent Org 26276.

### B: Urinary excretion of endogenous cortisol in the guinea pig.

In guinea piogs cortisol is endogenously produced, the basal level of which is approximately 120-150 µg/ml in plasma. Upon intravenous administration of Org 26276 at 5 mg/250 g, the amount of urinary cortisol excreted during 24 hours was determined and found to be increase 3-fold compared to the control group that did not receive Org 26276.

## Claims

1. Use of a cortisol-sequestering agent, having an association constant for cortisol of at least 30,000 M⁻¹, for the manufacture of a medicament for a treatment to lower cortisol levels in a patient.

2. The use according to claim 1, **characterised in that** the patient is having a disorder selected from the list of major depression, Cushing's syndrome, schizophrenia and severe anxiety disorders.

3. The use according to claim 2, **characterised in that** the disorder is major depression.

4. The use according to any one of claims 1-3, **characterised in that** the cortisol-sequestering agent is a cyclodextrin.

5. The use according to claim 4, **characterised in that** the cyclodextrin is a γ-cyclodextrin.
